Europäisches Patentamt

European Patent Office

Office européen des brevets

(11) Publication number: **0 421 966 A1**

# EUROPEAN PATENT APPLICATION

(21) Application number: **90850310.5**

(22) Date of filing: **19.09.90**

(51) Int. Cl.⁵: **A61F 2/02**

(30) Priority: **19.09.89 SE 8903099**

(43) Date of publication of application:
**10.04.91 Bulletin 91/15**

(84) Designated Contracting States:
**DE FR GB IT NL**

(71) Applicant: **RADI MEDICAL SYSTEMS AB**
**Palmbladsgatan 10**
**S-754 50 Uppsala(SE)**

(72) Inventor: **Ivancev, Krasnodar, MD, Oregon**
**Health Science Univ**
**Charles Dotter Inst. L-605, 3181 SW Sam**
**Jackson**
**Park Road, Portland, Oregon 97201(US)**
Inventor: **Lunderqvist, Anders**
**Svenska Vägen 48**
**S-222 39 Lund(SV)**

(74) Representative: **Svanfeldt, Hans-Ake et al**
**DR. LUDWIG BRANN PATENTBYRA AB Box**
**1344 Drottninggatan 7**
**S-751 43 Uppsala(SE)**

(54) **Embolisation guide wire.**

(57) A device for intravascular embolisation comprising a guide wire (3-6), known per se, having a tube (5), helix (4) provided at the distal end of said tube, a pull wire (3) extending through said helix and said tube and an expanding structure (1) provided at the distal end of said helix. The expanding structure is attached to the distal end of the pull wire.

FIGUR 1

EP 0 421 966 A1

## EMBOLISATION GUIDE WIRE

Background of the invention:

Embolisation means inter alia shutting off the blood supply to a blood vessel by blocking the vessel with a foreign material. If for example the vessel has a malformation and no operation is possible there is a way out by shutting off the blood supply (=to embolise) the vessel. Other applications may for example involve stopping the blood supply to a tumour, stopping a bleeding etc. Further applications for the embolisation guide wire in accordance with the invention involve for example sterilizing women by depositing embolisation material in the oviducts.

Today there are several methods to embolise blood vessels. Perhaps the most common method involves insertion of a helix in the vessel. However, this method is not suitable if the vessels are tiny. Another embolisation method is the use of a latex balloon of appropriate size which is inserted into the vessels. Still another method is to glue the vessel using a bucrylat based cement. Still another method involves injection of micro spheres through a catheter.

The existing methods have varying kinds of problems. The latex balloon method requires much training of the doctor until good results are achieved. It is difficult to position the latex ballon. There is also a risk that the x-ray contrast medium leaks out of the balloon, the balloon then slipping away from its desired position. Glueing is associated with a short hardening time in the order of about 3 seconds, this making it difficult to well control the embolisation of the vessel.

The present invention, an embolisation guide wire, offers a solution to two important problems. One problem refers to the distribution and this problem is solved by attaching an expanding structure at the top of a guide wire and the second problem is the administration of the embolising means. The solution to the administration problem is a proctective coating applied over the expanding structure. The protective coating will allow the administration of the embolisation material to the desired location before the structure starts to expand.

Varios embodiments of the invention will be described below in connection with the accompanying drawings wherein

Figure 1 is a cross-sectional view of an embolisation guide wire in accordance with the present invention,

Figure 2A is a perspective view of a second embodiment of the tip portion of the embolisation guide wire in accordance with the present invention,

Figure 2B is an end view of the tip portion shown in Figure 2A,

Figure 3A is a cross-sectional view of the tip portion of a third embodiment of the embolisation guide wire in accordance with the present invention, and

Figure 3B is a schematic perspective view of the tip portion of the embolisation guide wire in accordance with Figure 3A when the embolisation material has expanded.

Description of preferred embodiments

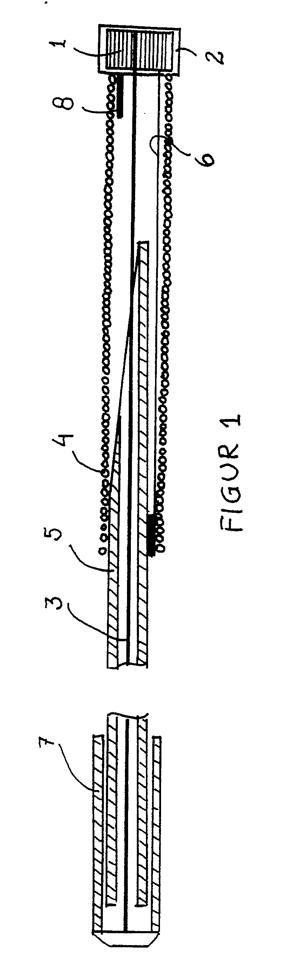

The proximal end of the embolisation guide wire shown in Figure 1 comprises an expanding structure 1 provided with a protective coating 2. The expanding structure is moulded on a pull wire 3 at the tip of the embolisation guide wire which further comprises a helix 4, a tube 5 and a safety wire 6. The pull wire extends through the guide wire and is attached to a pull sleeve 7 at the proximal end of the guide wire. At the tip portion of the guide wire there is a radiopaque marking 8 of the same lenght as the axial length of the structure 1. The expanding structure comprises a material such as a hydrophilic resin which expands upon contact with blood. The protective coating 2 is hydrophobic and is dissclved by a fat dissolving liquid. The protective coating is of fat.

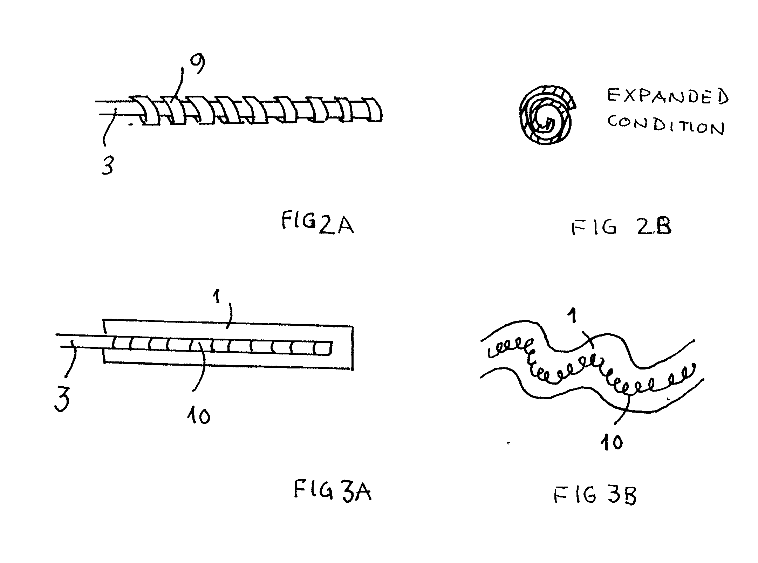

Expansion is augmented if the structure is provided in the form of a ribbon 9 which is wound into a helix. The length of the helix will elongate when the structure expands as is shown in figures 2A and 2B. Expansion is also augmented if the structure is moulded into a helix 10 which upon expansion will form another helix which embolises a larger lumen as is shown in Figures 3A and 3B.

The embolisation material is distributed in the following way:

1. The embolisation guide wire is moved and manipulated to its desired loaction using conventional guide wire practices and x-ray screening. The guide wire is housed in a catheter through which contrast medium is administered.
2. A liquid medium is injected through the catheter in order to dissolve the protective coating.
3. A plastic material expands until the material remains fixed to the vessel.
4. From the outside the doctor pulls the pull sleeve thus releasing the embolisation material from the guide wire.
5. The guide wire is withdrawn from the vessel.

## Advantages:

The big advantages with the system described above is that conventional guide wire-catheter practices can be used for introducing the embolisation material. The selective process also makes it possible to interrupt the insertion procedure and plenty of time is available for bringing the embolisation material to its desired location.

## Modifications:

The embolisation guide wire may be modified in many ways.

1. The expanding structure may also comprise a hydrophobic layer resistant to blood. After bringing the expanding structure to its desired location a liquid dissolving the hydrophobic layer is injected. As an alternative the expanding structure is of a nature that allows ampel time for moving and manipulating the guide wire to its desired position. In still another embodiment the protective coating 2 is of a material that is dissolved by the heat of the body after a predetermined period of time.

2. The guide wire can be so modified that its helix 4 extends all the way back to the proximal end of the guide wire.

## Claims

1. A device for intravascular embolisation **characterized by** a guide wire (3-6; 3, 10), known per se, comprising a tube (5), a helix (4; 10) provided at the distal end of said tube, a pull wire (3) extending through said helix and said tube and an expanding structure (1) provided at the distal end of said helix, attached to the distal end of said pull wire and having a protective coating which is inert to blood and dissolves by a heat and/or liquid treatment.

2. Device in accordance with claim 1, **characterized in that** said protective coating (2) comprises fat.

3. A device in accordance with claim 1 or 2, **characterized in that** said pull wire (3) extends through the tube from the distal end to the proximal end of said guide wire, that a pull sleeve (7) is provided at the proximal end of said guide wire, that the proximal end of the pull wire is attached to the pull sleeve, said pull wire, after expansion of said structure, being pulled out of said structure and said guide wire finally being withdrawn from the blood vessel so embolised.

4. A device in accordance with claim 3, **characterized in that** the expanding structure is a hydrophilis resin material.

5. A device in accordance with any of claims 1-4, **characterized in that** the expanding structure is in the form of a ribbon wound into a helix around the pull wire (3).

6. A device in accordance with any of claims 1-4, **characterized in that** the expanding structure is provided on a helix (10) made of metal, said metal helix expanding to a helix structure upon release of said pull wire.

7. A device in accordance with any of claims 1-6, **characterized in that** the expanding structure comprises a radiopaque material such as bismuth carbonate.

8. A device in accordance with any of claims 1-7, **characterized in that** said guide wire (3-6; 3, 10) is provided with a radiopaque marking (8) having the same length, as seen in the direction of said guide wire, as the length of the expanding structure (1) in its expanded condition.

FIGUR 1

EP 0 421 966 A1

FIG 2A

EXPANDED
CONDITION

FIG 2B

FIG 3A

FIG 3B

EP 0 421 966 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 832 055 (PALESTRANT) — — — | | A 61 F 2/02 |
| A | US-A-4 425 908 (SIMON) — — — | | |
| A | US-A-4 820 298 (LEEVEN et al.) — — — — — | | |

|  |  |
|---|---|
| | TECHNICAL FIELDS SEARCHED (Int. Cl.5) |
| | A 61 F<br>A 61 B<br>A 61 M |

The present search report has been drawn up for all claims

| Place of search | Date of completion of search | Examiner |
|---|---|---|
| The Hague | 15 November 90 | MIR Y GUILLEN V. |